# EUROPEAN PATENT APPLICATION

(11) **EP 1 310 247 A1**
(43) Date of publication of application: **14.05.2003**
(21) Application number: 03002581.1
(22) Date of filing: 04.11.1998
(51) Int. Cl.: A61K 31/137

(54) **(+)-ephedrine as a sympathomimetic drug**

(30) Priority: 14.11.1997 US 65572 P
(62) Divisional of application: 98956561.9
(71) Applicant: Warner-Lambert Company LLC, Morris Plains, New Jersey 07950 (US)
(72) Inventor: Booth, Anthony R., Chester, New Jersey 07930 (US); Sherman, William T., Henderson Ville, NC 28739 (US); Raven, Peter, Forth Worth, Texas 76107 (US); Caffrey, James L., Burleson, Texas 76028 (US); Yorio, Thomas, Burleson, Texas 76028 (US); Forster, Michael, Forth Worth, Texas 76133 (US); Gwirtz, Patricia, Forth Worth, Texas 76107 (US)
(74) Representative: Krohn, Sabine, Dr.

(57) **Abstract**

A pharmaceutical composition containing a therapeutically effective amount of (+)-ephedrine and a pharmaceutically aceptable carrier, wherein the composition is substantially free of (-)-ephedrine. The pharmaceutical composition in useful as a decongestant, appetite suppressant, and for treating other conditions often treated with sympathomimetic drugs. The (+)-ephedrine used in the present compositions and methods is substantially free of (-)-ephedrine, and of the adverse side effects associated with administration of (-)-ephedrine.

## Description

### FIELD OF THE INVENTION

The present invention is directed to pharmaceutical compositions which contain (+)-ephedrine and methods of using those compositions. The present (+)-ephedrine compositions may be used as decongestants, appetite suppressants, and for treating other conditions typically treated with sympathomimetic drugs. The pharmaceutical compositions of this invention are substantially free of (-)-ephedrine. As a result, the present compositions and methods of use have reduced adverse or undesirable side effects that are often associated with administration of (-)-ephedrine. Adverse side effects avoided by the present invention include stimulation of the central nervous system and cardiovascular system, and increased stimulation of the central nervous system when combined with an H₁ receptor antagonist, for example, an antihistamine.

### BACKGROUND OF THE INVENTION

Sympathomimetic drugs are structurally and pharmacologically related to amphetamine. They generally act by binding to, or activating, α- and β-adrenergic receptors resulting in vascular constriction, reduced blood flow and reduced secretion of fluids into the surrounding tissues. Such receptor binding generally decreases the amount of mucous secreted into nasal passages. Sympathomimetic drugs are therefore used to treat nasal congestion, allergies and colds. In addition, sympathomimetic drugs can influence the activity of the central nervous system, and are used as appetite suppressants; they are also known as mydriatic agents, meaning that they dilate the eye.

Compounds which have the same atomic composition and connectivity but are mirror images of each other are enantiomers. The prefixes *d* and *I*, or (+) and (-), identify the direction in which an enantiomer rotates light. The *d*- or (+) enantiomer is dextrorotatory. In contrast, the *I*- or (-) enantiomer is levorotatory. A mixture of (+) and (-) enantiomers is called a racemic mixture. As used herein, the term "enantiomer" means a single enantiomer which is substantially free of the opposite enantiomer and/or of any other diastereomer. Also as used herein, "substantially free" means that the ratio of (+)-enantiomer to (-)-enantiomer of at least 80:20, for example 85:15, and preferably 90:10, more preferably 95:5, and still more preferably, 99:1 or greater.

An alternative classification system for stereoisomers uses prefixes (S) and (R), based on the structural configuration of the chiral center rather than the optical activity of the compound.

Alpha-[1-(methylamino)ethyl]benzene-methanol is a molecule with two chiral centers. As such, this compound has four stereoisomers, occurring as two pairs of enantiomers. One pair of enantiomers is called "erythro," the other pair is called "threo." The erythro enantiomers of alpha-[1-(methylamino)ethyl]benzene-methanol are called (+)-ephedrine and (-)-ephedrine.

Some sympathomimetic compounds are known. For example, (+)-pseudoephedrine is a sympathomimetic compound which is sold as a decongestant. However, (+)-pseudoephedrine can readily be converted into the psychoactive drug, S-methamphetamine, by reduction of the hydroxyl group to hydrogen. Hence, this commonly-used decongestant has some undesirable features.

(-)-Ephedrine and the racemic mixture of (-)- and (+)-ephedrine have been used to treat nasal congestion. However, (-)-ephedrine and the racemic mixture of (-)- and (+)-ephedrine stimulate the central nervous system. 95 AMERICAN HOSPITAL FORMULATORY SERVICE 815. Accordingly, a need exists for a nasal decongestant which has the beneficial decongestant effects of (+)-pseudoephedrine and (-)-ephedrine, without their undesirable features or adverse side effects

### SUMMARY OF THE INVENTION

The invention features a pharmaceutical composition including a therapeutically effective amount of (+)-ephedrine and a pharmaceutically acceptable carrier, wherein the composition is substantially free of (-)-ephedrine, e.g., wherein the ratio of (+)-ephedrine to (-)-ephedrine is greater than 90:10, such as 95:5 or 99:1. In one aspect, the present composition substantially fails to induce in a patient an adverse side effect induced by the administration of another composition, such as a racemic mixture, including substantial amounts of (-)-ephedrine. According to the invention, the adverse side effect is reduced, milder, or essentially avoided. Adverse side effects include stimulation of the cardiovascular system (e.g., high blood pressure), stimulation of the central nervous system, and an interaction with another drug such as an H₁ receptor antagonist like triprolidine.

The disclosed composition can be formulated in concentrations and dosage units such that between 1 and 6 dosage units comprise an amount of ephedrine sufficient to reduce one or more physiological effects of histamine, to reduce nasal or bronchial congestion, to suppress the appetite, to reduce the symptoms of attention deficit hyperactivity disorder, or to activate an adrenergic receptor, or adrenergic receptor-mediated physiological response. Another aspect of the invention is a disclosed composition that further includes an H₁ receptor antagonist. One embodiment of the invention is a disclosed pharmaceutical composition, packaged with instructions for the self-administration of the composition for at least one of the following pharmacological activities: reducing nasal or bronchial congestion, suppressing the appetite, and reducing the symptoms of attention deficit hyperactivity disorder. Other indications that are appropriate for treatment with the disclosed compositions include those typically treated with sympathomimetic drugs.

The invention also features methods for treating the above-described conditions, wherein each method includes administering a therapeutically effective amount of a composition comprising (+)-ephedrine and a pharmaceutically acceptable carrier, the composition being substantially free of (-)-ephedrine. Such methods of treatment have advantages which include being characterized by a substantial reduction in the number and/or severity of one or more adverse side effects related to the administration of a composition which includes (-)-ephedrine, typically a racemic mixture of (+)- and (-)- enantiomers.

The invention also features methods further including administering an H₁ receptor antagonist to the patient. The H₁ receptor antagonist is selected, for example, from loratadine, descarboethoxy loratadine, fexofenadine, norastemizole, terfenadine, and ebastine, and is preferably triprolidine. The method can be practiced by formulating an H₁ receptor antagonist with (+)-ephedrine for simultaneous co-administration, or by administering separate dosages of an H₁ receptor antagonist before, during or after administering (+)-ephedrine. For example, separate dosages may be packaged together or segregated in two halves of a capsule or pill.

Until recently, sympathomimetic drugs were often sold as racemic mixtures because purification of one stereoisomer is expensive and time-consuming. Alternatively, a stereoisomer which can readily be isolated from nature is commercialized without testing whether or not a related stereoisomer may have greater efficacy and milder side effects. However, according to the present invention, one stereoisomer may interact more selectively than another with the receptors involved in sympathomimetic action. Isolation and use of the more selective stereoisomer may therefore reduce not only the required dosage, but one or more unwanted side effects.

The level of skill in this art is such that one could not reasonably predict the receptor binding specificity and subsequent pharmacological effects of one pure enantiomer even if one had the following information: 1) the receptor binding specificity and subsequent pharmacological effects of the other enantiomer; 2) the receptor binding specificity and subsequent pharmacological effects of the racemic mixture; or 3) the receptor binding specificity and subsequent pharmacological effects of both the racemic mixture and the other enantiomer.

Other aspects and advantages of the invention are described in the following description, examples, and appended claims.

### BRIEF DESCRIPTION OF THE DRAWINGS

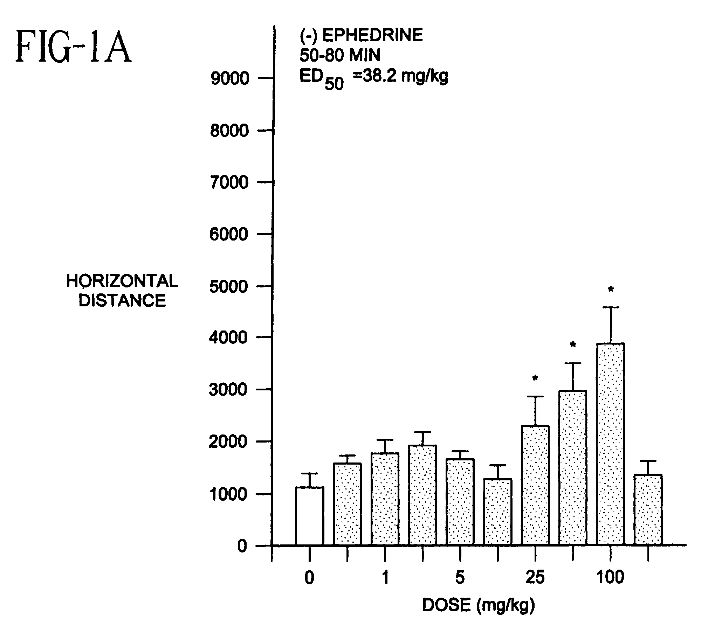

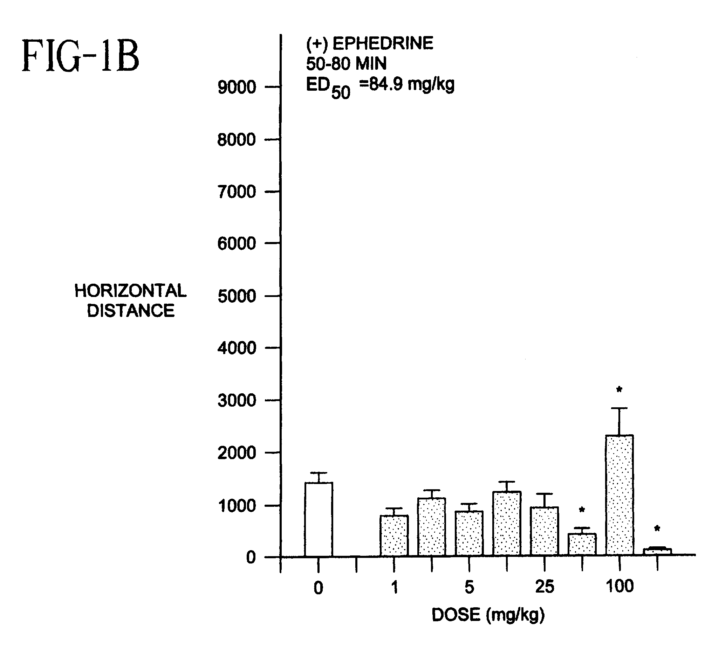

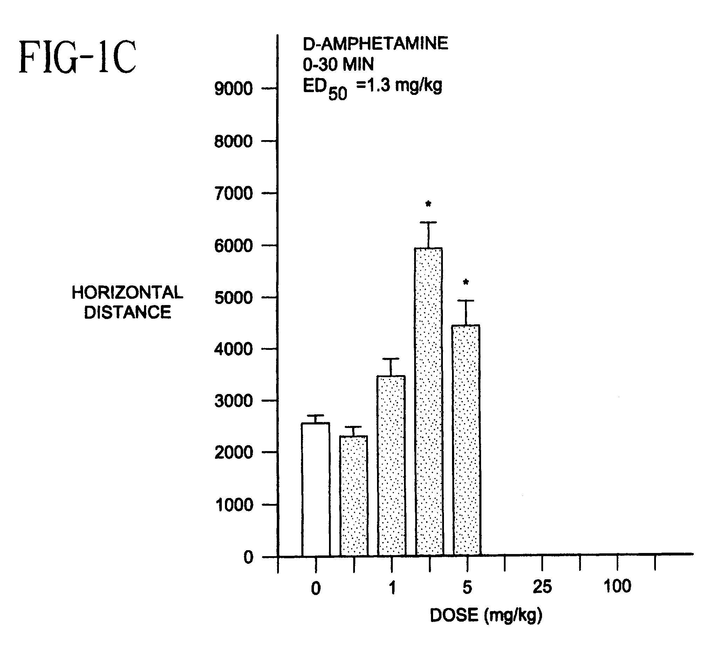

Figure 1 is a bar graph showing locomotor stimulant dose-response effects of (+)-ephedrine and (-)-ephedrine, and *d*-amphetamine at the peak response time. Each panel shows the mean horizontal distance (cm/10 minutes) traveled following saline (open bars) or doses (shaded bars) of the test compound for the 30-minute time period in which peak stimulant effects occurred. The standard error (S.E.) of the response at each dosage is indicated with an error bar. Maximal stimulant effects were estimated from a logistic peak function fit to the data. ED₅₀ values provided refer to the dosage resulting in ½ maximal stimulant effect as interpolated from a linear regression on the ascending limb of the dose-effect curve.

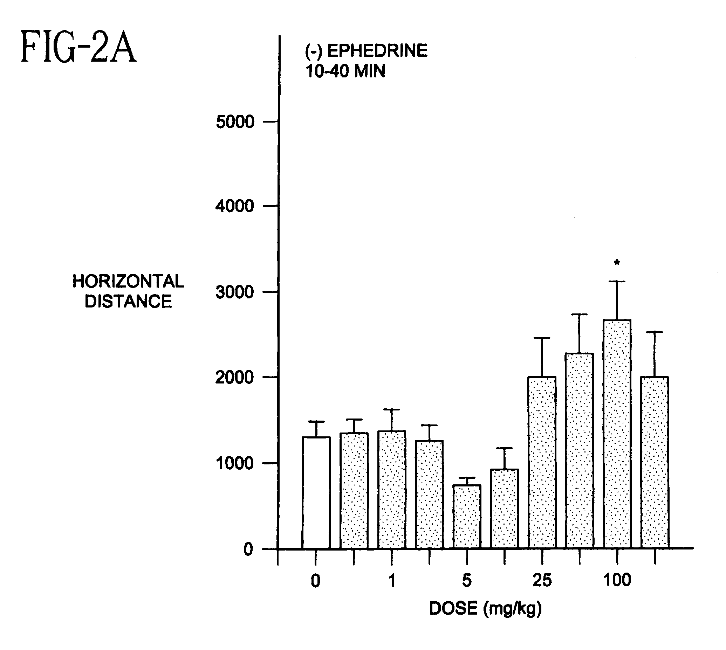

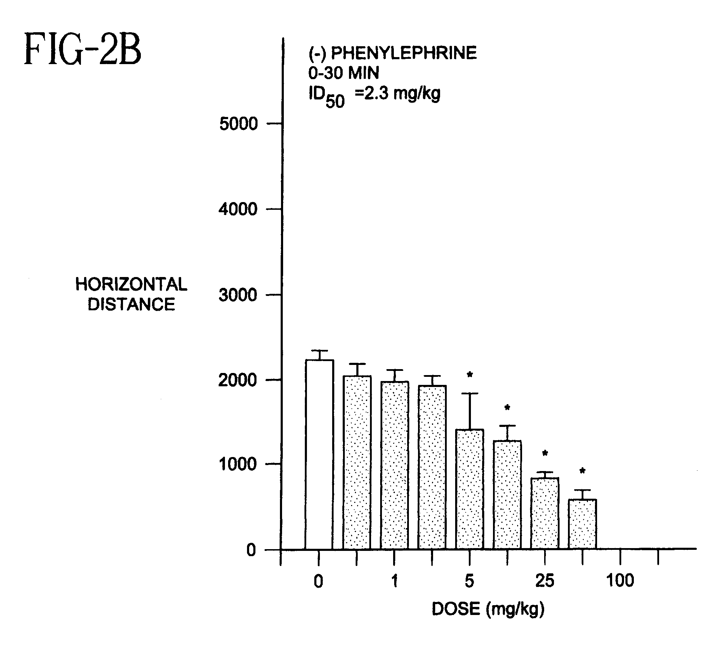

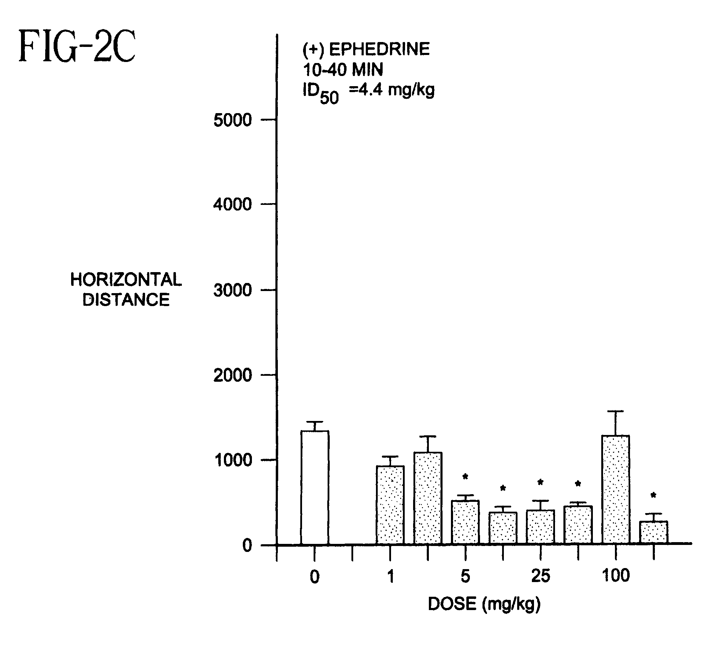

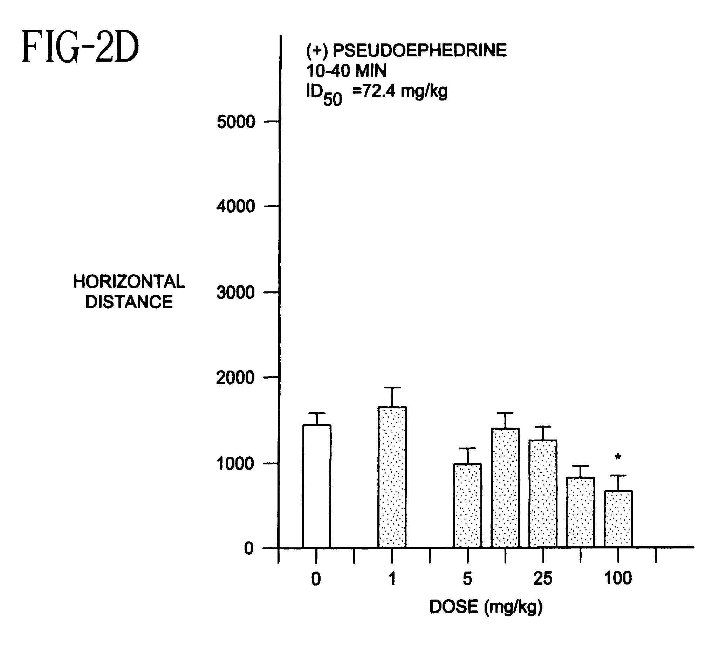

Figure 2 is a bar graph showing locomotor depressant dose-response effects for (+)-ephedrine, (-)-ephedrine, (+)-pseudoephedrine and (-)-phenylephrine. Each panel shows the mean horizontal distance (cm/10 minutes) traveled following saline (open bars) or doses (shaded bars) of the test compound for the 30 minute time period in which peak depressant effects occurred. The standard error (S.E.) of the response at each dosage is indicated with an error bar. The ID₅₀ values refer to the dosage resulting in ½ maximal depressant effect as interpolated from a linear regression on the descending limb of the dose-effect curve, with zero cm/10 minutes considered to be maximal depression.

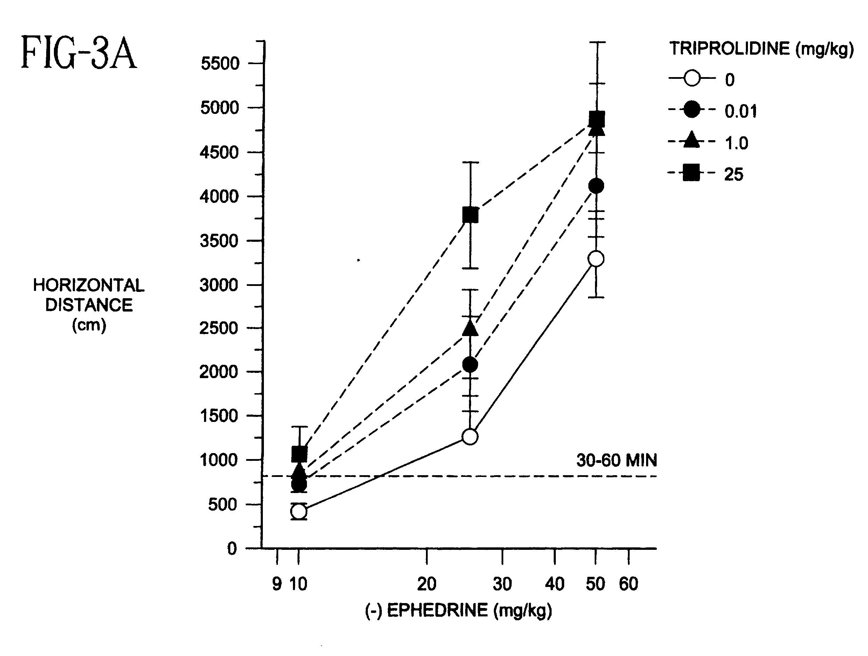

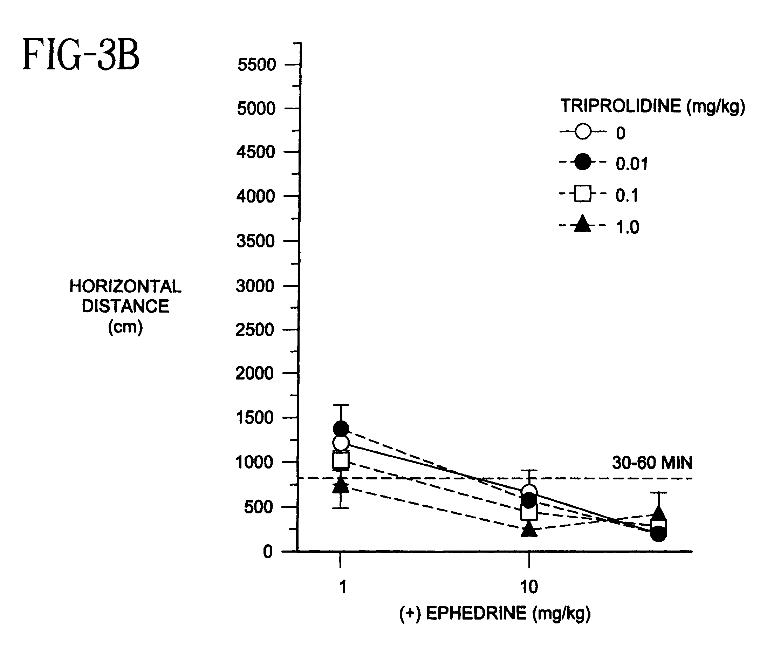

Figure 3 depicts the interactive effects of (-)- and (+)-ephedrine with the antihistamine triprolidine. In the left panel, the mean horizontal distance traveled as a function of (-)-ephedrine is provided at 20 minutes following pretreatment with triprolidine (0 to 25 mg/kg). The standard error (S.E.) of the response at each dosage is indicated with an error bar. In the right panel, the mean horizontal distance traveled as a function of (+)-ephedrine is provided at 20 minutes following triprolidine pretreatment (0.01 to 1 mg/kg). The horizontal dashed lines indicate the mean horizontal distance traveled by the control group receiving only saline injections.

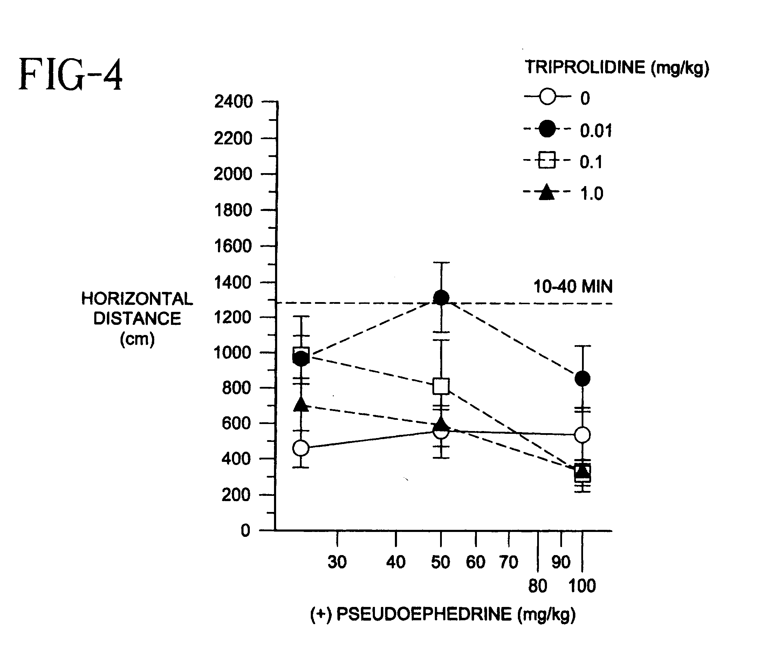

Figure 4 depicts the interactive effects of (+)-pseudoephedrine with the antihistamine triprolidine. The mean horizontal distance traveled as a function of (+)-pseudoephedrine dosage is provided at 20 minutes following pre-treatmentwith triprolidine(0.01 to 1 mg/kg). The horizontal dashed lines indicate the mean horizontal distance traveled by the control group receiving only saline injections. The standard error (S.E.) of the response at each dosage is indicated with an error bar.

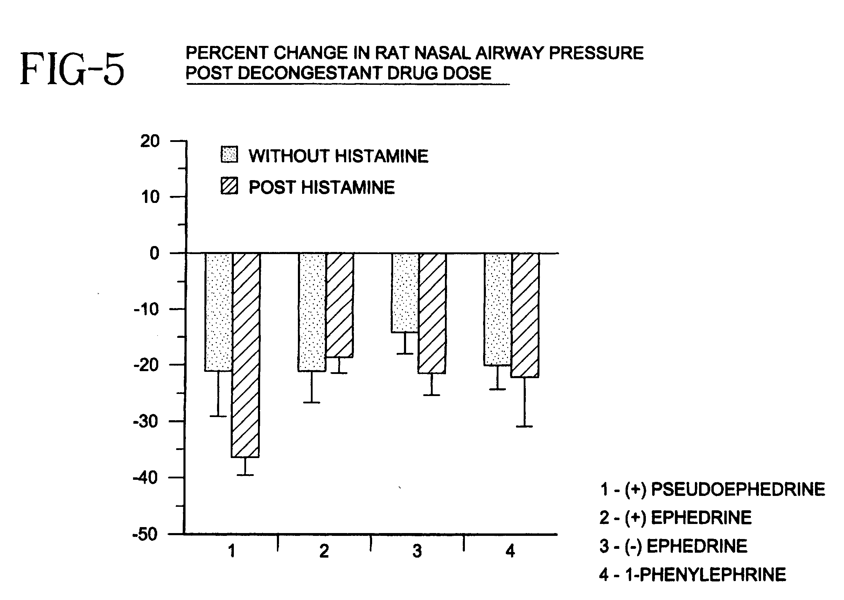

Figure 5 shows the percent change in nasal airway pressure as percent of control and as percent of post-histamine levels associated with (+)-ephedrine, (-)-ephedrine, (-)-phenylephrine and (+)-pseudoephedrine. The standard error (S.E.) of the response at each dosage is indicated with an error bar.

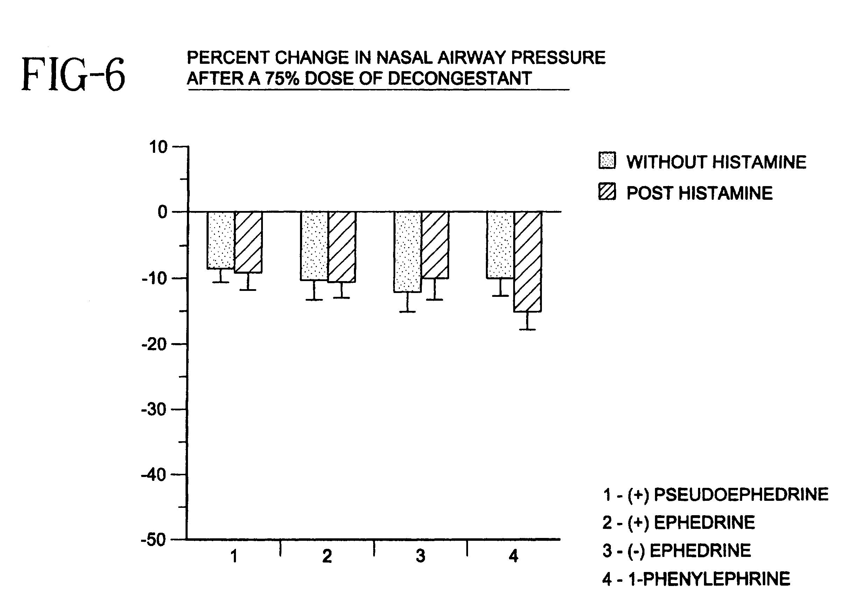

Figure 6 shows the percent change in nasal airway pressure after a "75% dose" of (+)-ephedrine, (-)-ephedrine, (-)-phenylephrine and (+)-pseudoephedrine. The "75%" dose is 75% of the dose found to increase the mean arterial blood pressure 10%.

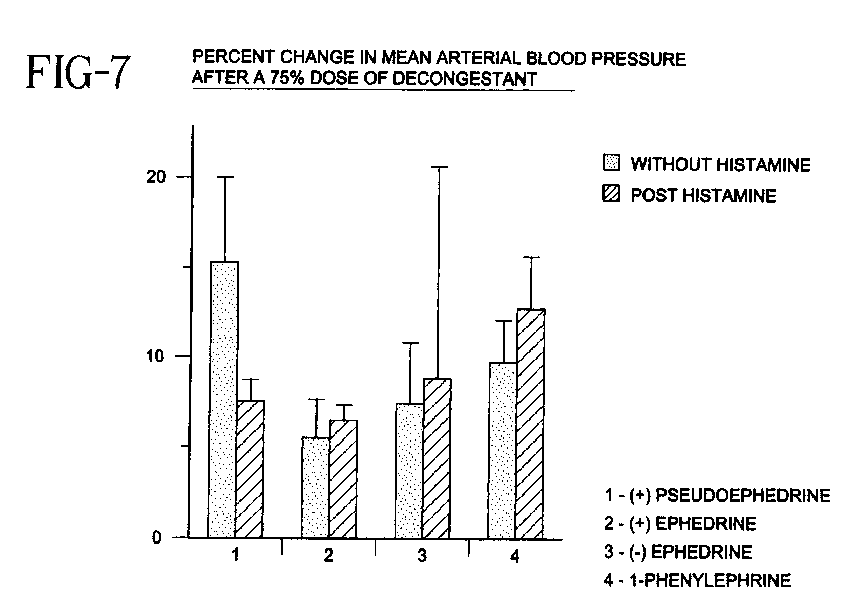

Figure 7 shows the percent change in mean arterial blood pressure after a "75%" dose of (+)-ephedrine, (-)-ephedrine, (-)-phenylephrine and (+)-pseudoephedrine. The "75%" does is 75% of the dosage found to increase the mean arterial blood pressure by 10%.

### DETAILED DESCRIPTION OF THE INVENTION

The present invention is directed to a pharmaceutical composition of (+)-ephedrine and a pharmaceutically acceptable carrier, wherein the pharmaceutical composition is substantially free of (-)-ephedrine. The pharmaceutical composition includes (+)-ephedrine in a therapeutic dosage suitable for treating nasal and bronchial congestion, obesity, attention deficit hyperactivity disorder (ADHD), as well as for acting as an appetite suppressant and for treating other conditions typically treated with sympathomimetic drugs.

Both (+)- and (-)-ephedrine are erythro-stereoisomers of α-[1-(methylamino) ethyl]benzene-methanol. The structures of the free amines of (+)-ephedrine and (-)-ephedrine are as follows:

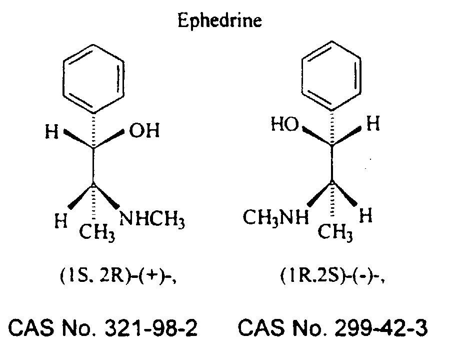

The term "substantially free" of (-)-ephedrine, as used herein, means that the pharmaceutical composition contains at least 80% (+)-ephedrine and 20% or less (-)-ephedrine, based on the weight percentage of (+)-ephedrine to (-)-ephedrine. In a preferred embodiment, "substantially free" of (-)-ephedrine means that the composition contains at least 90% (+)-ephedrine and 10% or less (-)-ephedrine. In a more preferred embodiment, "substantially free" of (-)-ephedrine means that the composition contains at least 95% (+)-ephedrine and 5% or less (-)-ephedrine based upon the weight percentage of (+)-ephedrine and (-)-ephedrine. Still more preferred is an embodiment wherein the pharmaceutical composition contains 99% (+)-ephedrine based upon the weight percentage of (+)-ephedrine and (-)-ephedrine.

According to the present invention, the present composition of (+)-ephedrine is substantially free of adverse side effects related to the administration of (-)-ephedrine. Side effects associated with administration of (-)-ephedrine include stimulation of the central nervous system, stimulation of the cardiovascular system, and drug interactions. Such drug interactions include increased stimulation of the central nervous system when (-)-ephedrine is administered with other drugs, such as an H₁ receptor antagonist (e.g., an antihistamine). Administration of the present compositions of (+)-ephedrine reduces or avoids such side effects. It is also believed that administration of (+)-ephedrine, substantially free of (-)-ephedrine, would produce fewer side effects relative to administration of the racemic mixture of (+)- and (-)-ephedrine.

Thus, according to the present invention, (+)-ephedrine does not interact with other drugs such as H₁ receptor antagonists, including antihistamines. In particular, co-administration of (+)-ephedrine with the antihistamine triprolidine does not give rise to any significant central nervous system stimulation or depression. Due to the lack of such drug interaction, supplementary active ingredients, such as additional antihistamines and decongestants, can be incorporated into the present compositions. The amount of the antihistamine present in the pharmaceutical composition will depend upon the particular antihistamine used.

Antihistamines which can be incorporated into the present compositions include, for example, alkylamines, aminoalkylethers, ethylenediamines, piperazines and tricyclic antihistamines. Alkylamine antihistamines include acrivastine, bamipine, brompheniramine, chlorpheniramine, dimethindene, Metron S, pheniramine, pyrrobutamine, thenaldine, tolpropamine and triprolidine. Aminoalkylether antihistamines include bietanautine, bromodiphenhydramine, carbinoxamine, clemastine, diphenhydramine, diphenylpyraline, doxylamine, embramine, medrylamine, moxastine, p-methyldiphenhydramine, orphenadrine, phenyltoloxamine and setastine. Ethylenediamine antihistamines include alloclamide, chloropyramine, chlorothen, histapyrrodine, methafurylene, methaphenilene, methapyrilene, pyrilamine, talastine, thenyldiamine, thonzylaminehydrochloride, tripelennamine, and zolamine. Piperazine antihistamines include cetirizine, chlorcyclizine, cinnarizine, clocinizine and hydroxyzine. Tricyclic antihistamines include ahistan, azatadine, chlobenzepam, cyproheptadine, deptropine, etymemazine, fenethazine, descarboethoxy loratadine, N-hydroxyethylpromethazine chloride, isopromethazine, isothipendyl, loratadine, mequitazine, promethazine, and thiazinamium methyl sulfate. Other antihistamines contemplated by the present invention include antazoline, norastemizole, azelastine, cetoxime, clemizole, astemizole, clobenztropine, ebastine emedastine, epinastine, fexofenadine, levocabastine, mebhydroline, phenindamine, terfenadine, and tritoqualine.

Preferred antihistamines include: acrivastine; astemizole; brompheniramine; chlorpheniramine; descarboethoxy loratadine diphenhydramine; loratadine; norastemizole; terfenadine; terfenadine carboxylate; and triprolidine.

(+)-Ephedrine may be prepared by known procedures or obtained commercially. Methods for separating the stereoisomers present in a racemic mixture are well-known to the skilled artisan. The present invention also provides pharmaceutically acceptable salts of (+)-ephedrine. For example, (+)-ephedrine can be provided as a hydrochloride, bitartrate, tannate, sulfate, stearate, citrate or any other pharmaceutically acceptable salt. Methods of making such pharmaceutical salts of (+)-ephedrine are readily available to one of ordinary skill in the art.

As used herein, "pharmaceutically acceptable carrier" includes any and all solvents, dispersion media, coatings, antibacterial and antifungal agents, isotonic and absorption delaying agents, sweeteners and the like. The pharmaceutically acceptable carriers may be prepared from a wide range of materials including, but not limited to, diluents, binders and adhesives, lubricants, disintegrants, coloring agents, bulking agents, flavoring agents, sweetening agents and miscellaneous materials such as buffers and absorbents that may be needed in order to prepare a particular therapeutic composition. The use of such media and agents with pharmaceutically active substances is well known in the art. Except insofar as any conventional media or agent is incompatible with the active ingredient, its use in the therapeutic compositions is contemplated. Supplementaryactive ingredients can also be incorporated into the compositions.

The present invention further contemplates a method of using (+)-ephedrine compositions for treating colds, nasal congestion, bronchial congestion, allergies, and obesity, as well as for providing appetite suppression and for treating other conditions typically treated with sympathomimetic drugs. Thus, in one embodiment the present invention is directed to a method of relieving nasal and bronchial congestion which comprises administering a therapeutically effective amount of (+)-ephedrine, wherein the (+)-ephedrine is substantiallyfree of (-)-ephedrine. Administration of a therapeutically effective amount of (+)-ephedrine which is substantially free of (-)-ephedrine reduces many of the side effects that are observed with administration of a composition including (-)-ephedrine. Such side effects include stimulation of the central nervous system, stimulation of the cardiovascular system and drug interactions including increased stimulation of the central nervous system and the cardiovascularsystem when administered with other drugs, for example, an H₁ receptor antagonist.

According to the present invention, a therapeutically effective amount of (+)-ephedrine is an amount sufficient to relieve one or more symptoms of a condition which can be treated by a sympathomimetic drug. In one embodiment, such an amount is an amount of (+)-ephedrine sufficient to bind or activate an adrenergic receptor, for example, an α- or a β-adrenergic receptor. When administered in such an amount, (+)-ephedrine can, for example, stimulate an adrenergic receptor mediated response. When the condition is nasal congestion, the therapeutically effective amount is the amount needed to reduce nasal congestion. When bronchial congestion is the condition, the therapeutically effective amount is the amount needed to reduce bronchial congestion or to provide bronchodilation. When inflammation and/or allergic reaction is the condition, the therapeutically effective amount is the amount needed to counteract the physiological effects of histamine. Preferably, such a therapeutically effective amount also produces fewer or milder side effects than are observed upon administration of (-)-ephedrine, or a racemic mixture of (+)- and (-)-ephedrine. The skilled artisan can readily determine the necessary therapeuticallyeffective amounts for treating these conditions, particularly in light of the teachings provided herein.

The pharmaceutical compositions of the present invention contain (+)-ephedrine in a therapeutically effective amount that is sufficient to provide decongestion, bronchodilation, suppress the appetite antagonize the effects of histamine, or treat attention deficit hyperactivity disorder, while having fewer or milder side effects than would similar doses of (-)-ephedrine or a racemic mixture of (+)- and (-)- ephedrine. Such a therapeutically effective amount would be about 0.1 micrograms (µg) to about 50 milligrams (mg) per kilogram of body weight, and preferably about 1 µg to about 10 mg per kg of body weight. More preferably, the dosage can range from about 10 µg to about 5 mg per kg of body weight. Even more preferably, the dosage can range from about 50 µg to about 500 µg per kg of body weight. Dosages can be readily determined by one of ordinary skill in the art and can be readily formulated into the subject pharmaceutical compositions. For example, one of skill in the art may determine a therapeutically effective amount of the present compositions by determining the amount of (+)-ephedrine sufficient to bind or activate an α-adrenergic receptor. The teachings of the present invention, including the methodologies and the data provided hereinbelow (Examples 1-3), further guide one of skill in the art to properly formulating compositions with a therapeutically effective amount of (+)-ephedrine. The appetite-suppressing properties of the disclosed compositions can be demonstrated,for example, by use in animal models known to those in the art, such as those described in J.L. Zelger and E.A. Carlini, 12 PHARMACOLOGY, BIOCHEMISTRY & BEHAVIOR 701-05 (1980).

The subject (+)-ephedrine may be administered by any convenient route. For example, (+)-ephedrine may be inhaled, ingested, topically applied or parenterally injected. The subject (+)-ephedrine may be incorporated into a cream, solution or suspension for topical administration. (+)-Ephedrine is preferably inhaled or administered orally or topically.

The present invention will now be further explained in the following examples. However, the present invention should not be construed as being so limited. Unless otherwise indicated, all parts, percentages and the like are by weight.

### EXAMPLE 1

### α-Adrenergic and β-Adrenergic Receptor Binding Studies

Many physiological processes are mediated by the binding of chemical compounds to α₁, α₂ and β receptors. For example, many compounds which reduce nasal congestion bind to α₁ and α₂ receptors and some reduce bronchial congestion by binding to β receptors. Accordingly, a compound that binds to α₁, α₂ and/or β₂ receptors may be an effective nasal or bronchial decongestant.

More specifically, α₂ adrenergic receptors, concentrated on precapillary arterioles in the nasal mucosa, induce arteriolar vasoconstriction when activated by a sympathomimetic compound. Such vasoconstriction decreases blood flow through those vessels and reduces excess extracellular fluid associated with nasal congestion and a runny nose. On the other hand, α₁ adrenergic receptors are concentrated on postcapillary venules in the nasal mucosa. Binding to α₁ receptors induces venoconstriction, which also reduces nasal congestion.

Compounds that bind to β₂ receptors may also help relieve the symptoms of nasal congestion because β₂ receptor binding is related to increased bronchodilation and reduced airway resistance.

The binding of (+)-ephedrine to α₁, α₂ and β₂ receptors was compared to that of (+)-pseudoephedrine, (-)-phenylephrine and (-)-ephedrine. (+)-Pseudoephedrine is a known decongestant sold under the trade name SUDAFED®(Warner-LambertCo., Morris Plains, NJ). (-)-Phenylephrine(NEO-SYNEPHRINE®, Sanofi, East Hanover, NJ) is also known to be an decongestant.

### Methods: Membrane Preparations.

PULMONARY ALPHA-1 AND BETA-2 RECEPTORS. The lungs of mongrel dogs were separated from cartilaginous airways and major blood vessels, weighed, chopped and placed into 10 volumes of ice-cold buffered sucrose (50 mM Tris-HCI, pH 7.4, 1 mM EGTA, 0.32 M sucrose). The tissue was then homogenized in a type PT10/35, size 89/PTA 20S, Polytron tissue homogenizer (Kinematica, Westbury, NY). The homogenate was filtered through two layers of cheesecloth, and the filtrate was dounced three times using a 55 ml Con-Torque Potter homogenizer (Eberbach Corporation, Ann Arbor, Ml). The dounced filtrate was centrifuged at 1000 x g for 15 minutes at 4°C. The supernatant was re-centrifuged at 30,000 x g for 30 minutes at 4°C and the resulting pellet was washed and resuspended in 10 volumes of Tris buffer (50 mM Tris HCL, pH 7.4, 1 mM EGTA) and incubated at 37°C for 30 minutes in a shaking water bath. The suspension was centrifuged at 4°C at 30,000 x g for 30 minutes and the resulting pellet washed in 10 volumes of Tris buffer for a total of three additional centrifugationsand washes. The final pellet was resuspended in 0.5 volume of 50 mM Tris HCI, pH 7.4, 1 mM EGTA, 25 mM MgCl₂. Protein concentration was determined by the Lowry method and the final suspension was adjusted to 10 mg of protein/ml, aliquoted and stored at -90°C. Particulates were also prepared for β₂ receptors using the identical procedure except the final protein concentration was adjusted to 0.1 mg/ml.

BRAIN ALPHA-2 RECEPTORS: Membranes of mongrel dogs were harvested from the canine frontal cortex and prepared as described above with respect to the lungs, except that the final membrane protein concentration was adjusted to 0.5 mg/ml.

### Binding Assays:

ALPHA-1 BINDING, ³H-PRAZOCIN: Canine lung membrane preparations (500 µg protein/100 µl) were incubated with ³H-Prazocin (77.9 Ci/mmol) for 60 minutes at 25°C in a final volume of 0.25 ml of buffer (50 mM Tris-HCI, 1 mM EGTA, pH 7.4). Nonspecific binding was determined for each concentration point in separate incubations using 10 µM phentolamine. Each experimental point was determined in triplicate. The final concentration of ³H-Prazocin was 0.7-1.1 nM in competition studies and between 0.1 and 10 nM in saturation experiments. All binding assay incubations were terminated by rapid dilution with 2 ml of ice-cold wash buffer (50 mM Tris-HCI, pH 7.4) and filtration through FP-100 Whatman GF/B fired filters (Biomedical Research & Development Lab, Gaithersburg, MD) using a Brandel receptor-binding harvester. The filters were washed twice more with 4 ml of wash buffer and then added to 6 ml Cytoscint (ICN, Costa Mesa CA) for liquid scintillation counting (Barnes et al., 23 MOL. PHARMACOL. 570-75 (1983)). In all experiments, less than 17% of the added radioligand was bound, and specific binding was about 65-70% of total binding.

ALPHA-2 BINDING, P-¹²⁵IODOCLONIDINE. Canine brain membranes (50 µg protein/100 µl) were incubated with p-iodoclonidine (2200 Ci/mmol) for 120 minutes at 25°C in a final volume of 0.25 ml. Nonspecific binding was determined in separate incubations in the presence of 10 µM phentolamine. The final concentration of p-iodoclonidine was 44-45 pM in competition studies and between 50 pM and 10 nM in saturation experiments. Bound and free p-¹²⁵ iodoclonidine were separated and the bound quantitated as described below for the ICYP assays. An average of 6% of radioligand was bound, and specific binding was about 91% of total binding.

BETA-2 BINDING, ¹²⁵IODOCYANOPINDOLOL (¹²⁵ ICYP). Canine lung membranes (10 µg protein/100 µl) were incubated with ¹²⁵ICYP (2200 Ci/mmol) for 110 minutes at 30°C in a final volume of 0.25 ml. Nonspecific binding was determined in separate incubations in the presence of 2 µM *d,l*-propranolol. Each experimental point was determined in triplicate. The final concentration of ¹²⁵ICYP was 8-12 pM in competition studies and between 2 and 200 pM in saturation experiments. Incubations were terminated as described above for the α₁ assays. Filters were placed into polyethylene tubes and the bound ligand was determined by gamma spectrometry(Sano et al., 52 LIFE SCIENCES 1063-70 (1993)). An average of 27% of radio ligand was bound, and specific binding was about 90% of total binding.

All data were analyzed with the aid of microcomputer nonlinear curve fitting programs (PRISM 2.0, Graphpad Software, San Diego CA).

### Results:

The receptors resident in each of the three membrane preparations were evaluated by standard saturation analysis following the addition of increasing concentrations of the appropriate radioligand. In the case of the α₁- and β₂-assays, the mathematical analysis was consistent with a one site fit. The α2-receptor analysis was best fit as two sites, one high and one low affinity.

The radioligand added for subsequent α₂-displacement assays was adjusted to evaluate only the high affinity receptor. Contributions from p-iodoclonidine binding to imidazoline receptors in the α₂- displacement assay was evaluated with epinephrine. Epinephrine easily displaced all bound p-iodoclonidine which indicates that at the concentrations employed, p-iodoclonidine labeled few if any imidazoline receptors. Similarly, with the β₂-assay, contributions from the binding of ICYP to β₁ sites was evaluated with the β₁-selective antagonist, atenolol. Atenolol was largely ineffective in displacing ICYP from pulmonary membranes indicating little if any β₁ binding within the assay. All subsequent analyses with displacementby individual test compounds used the K_{d} determined from the saturation analysis since it is generally considered a more reliable estimate of the true equilibrium dissociation constant

Table 1 provides the binding characteristics of the α₁-receptors in the membrane preparation for prazocin. The K_{d} is the apparent equilibrium dissociation constant for prazocin. The Bₘₐₓ is the number of α₁-receptor binding sites for prazocin in this membrane preparation expressed as femtomoles per mg protein.

**TABLE 1**

| α₁-Receptor Binding Characteristics (canine lung membranes) | |
|---|---|
| Measure | Summary |
| Scatchard Analysis | |
| K_{d} | 0.84 nM |
| Bₘₐₓ | 55 |

| Saturation Analysis | |
|---|---|
| K_{d} | 0.73 nM |
| Bₘₐₓ | 53 |

Table 2 provides the binding characteristics of the α₂-receptors in the membrane preparation for p-iodoclonidine. The K_{d} is the apparent equilibrium dissociation constant for p-iodoclonidine. The Bₘₐₓ is the number of α₂-receptor binding sites for p-iodoclonidine in this membrane preparation expressed as femtomoles per mg protein. Note that the two site data from the Saturation Analysis is more reliable than the Scatchard Analysis because the Scatchard Analysis assumes only one site. In order to obtain both values from the Scatchard plots, the points in the transition zone were arbitrarily divided and assigned to high and low affinity plots.

**TABLE 2**

| α₂-Receptor Binding Characteristics (canine cerebral cortex membranes) | |
|---|---|
| Measure | Summary |
| Scatchard Analysis | |
| K_{d 1} (high affinity) | 0.15 nM |
| K_{d 2} (low affinity) | 0.87 nM |
| Bₘₐₓ₁ (high affinity) | 67 |
| Bₘₐₓ₂ (low affinity) | 120 |

| Saturation Analysis | |
|---|---|
| K_{d 1} (high affinity) | 0.15 nM |
| K_{d 2} (high affinity) | 3.01 nM |
| B_{max 1} (high affinity) | 57 |
| Bₘₐₓ₂ (low affinity) | 73 |

Table 3 provides the binding characteristics of the β₂-receptors in the membrane preparation for ¹²⁵iodocyanopindolol (ICYP). The K_{d} is the apparent equilibrium dissociation constantfor ICYP. The Bₘₐₓ is the number of β₂-receptor binding sites for ICYP in this membrane preparation expressed as femtomoles per mg protein.

**TABLE 3**

| β₂-Receptor Binding characteristics (canine lung membranes) | | | |
|---|---|---|---|
| Measure | Run 1 | Run 2 | Summary |
| Scatchard Analysis | | | |
| K_{d} | 9.9 pM | 7.8 pM | 8.9 pM |
| Bₘₐₓ | 150 | 139 | 145 |

| Saturation Analysis | | | |
|---|---|---|---|
| K_{d} | 9.6 pM | 9.3 pM | 9.5 pM |
| Bₘₐₓ | 149 | 142 | 146 |

Table 4 below shows the concentration (µM) of the test drug required to inhibit 50% of specific prazocin, p-iodoclonidine or ICYP binding (IC₅₀). The ratios of the apparent dissociation constants (Kᵢ) are also provided, and were calculated for each compound based on the relationship Kᵢ = IC₅₀ / 1 + I/K_{d} (where I is the concentration of tracer added and the K_{d} is the equilibrium dissociation constant empirically determined for the receptor population using the saturation analysis values). The Kᵢ between receptors is provided for each compound to express the relative preference of each for the receptor in question. For example, ratios near one indicate little preference of the compound for either receptor. Higher ratios indicate a preference for the receptor in the denominator and numbers less than one indicate a preference for the receptor in the numerator. The data shown in Table 4 is the mean data for IC₅₀ and Kᵢ collected from several test samples for each compound.

**TABLE 4**

| | **Alpha-1** | | **Alpha-2** | | **Beta-2** | | **K**_{**i**}**-Ratio** | | |
|---|---|---|---|---|---|---|---|---|---|
| **Drugs** | **IC**_{**50**} | **K**_{**i**} | **IC**_{**50**} | **K**_{**i**} | **IC**_{**50**} | **K**_{**i**} | **α1/α2** | **α1/β2** | **β2/α2** |
| (+)-pseudoephedrine | 691 | 299 | 28 | 21 | 502 | 220 | 14.2 | 1.35 | 10.5 |
| (-)-phenylephrine | 7 | 3 | 0.02 | 0.02 | 10 | 5 | 200 | 0.60 | 333.3 |
| (-)-ephednne | 109 | 47 | 0.77 | 0.59 | 12 | 5 | 79.7 | 9.40 | 8.47 |
| (+)-ephedrine | 92 | 40 | 31 | 24 | 1361 | 614 | 1.67 | 0.07 | 25.6 |

### EXAMPLE 2

### Effect Of (+)-Ephedrine On The Central Nervous System

Many sympathomimetics stimulate the central nervous system, which is a reason that many decongestants have undesirable side effects like insomnia. The following testing shows the degree of central nervous system stimulation for (+)-ephedrine relative to (+)-pseudoephedrine, (-)-phenylephrine, (-)-ephedrine and a control, (+)-amphetamine.

Moreover, decongestants are often sold in combination with other active ingredients (e.g., Claritin-D® made by Schering, Kenilworth, NJ and Seldane-D® made by Hoechst Marion Roussel, Kansas City, MO). In products containing two or more active ingredients, interactions between the active ingredients are undesirable. Also in this Example, the extent of (+)-ephedrine interaction with a known antihistamine, triprolidine, was observed and compared to any such interaction between triprolidine and (+)-pseudoephedrine, (-)-phenylephrine and (-)-ephedrine.

### Methods:

### Animals

Male Swiss-Webster mice (HSD ND, Harlan Sprague Dawley, Houston, TX), aged 2-3 months, were used in these studies. Each dose group consisted of 8 mice (total mice involved were 1,764). The mice were housed 2 to 4 per cage in 30.4 x 22.9 x 15.2 cm clear polycarbonate cages with food and water available *ad libitum* for at least one week prior to locomotor activity testing. The colony was maintained at 23±1°C, on a normal light-dark cycle beginning at 0700 hr. All testing took place during the light portion of the light-dark cycle.

### Apparatus

Horizontal (forward movement) locomotor activity was measured using a standardized, optical activity monitoring system (Model KXYZCM, Omnitech Electronics, Columbus, OH). Activity was monitored in forty 40.5 x 40.5 x 30.5 cm clear acrylic chambers that were housed in sets of two within larger sound-attenuating chambers. A panel of 16 infrared beams and corresponding photodetectors were spaced 2 cm apart along the sides and 2.4 cm above the floor of each activity chamber. A 7.5-W incandescent light above each chamber provided dim illumination via a rheostat set to 20% of full scale. Fans provided an 80-dB ambient noise level within the chamber.

### Drugs

(+)-Pseudoephedrine,(-)-phenylephrine,(-)-ephedrine, (+)-ephedrineand (+)-amphetaminewere obtained from Sigma Chemical Company (St. Louis, MO). Triprolidine HCl was obtained from Research Biochemicals International, (Natick, MA). All compounds were dissolved in 0.9% saline and injected i.p. in a volume of 10 ml/kg body weight.

### Procedure

*Locomotor stimulant effects.* In these studies, mice were placed in the activity testing chambers immediately following injection of saline or a dose of one of the test compounds ranging from 0.1 mg/kg to 250 mg/kg. (+)-Amphetamine was used as a positive control. The total horizontal distance traversed (cm) was recorded at 10 minute intervals for a 2-hour session. Separate groups of 8 mice were assigned to each dose or saline group, and dose-effect testing continued for each compound until maximal stimulant or depressant effects could be estimated. A separate control group was tested along with each compound.

For compounds with significant stimulant effects, the potency and efficacy were estimated for the 30-minute time period in which maximal stimulant effects were observed at the lowest dose. Using TableCurve 2D v2.03 (Jandel Scientific, San Rafael, CA) the mean average total distance traversed (cm/10 min) for that period was fit to a 3-parameter logistic peak function of log₁₀ dose (with the constant set to the mean of the saline group), and the maximum effect estimated from the resulting curve. The ED₅₀ (dose producing ½ maximal stimulant activity) was estimated from a linear regression against log₁₀ dose of the ascending portion of the dose-effect curve. The stimulant efficacy was the peak effect of the compound (cm/10 min) as estimated from the logistic peak function, minus the mean control distance traveled (cm/10 min), and was expressed for each stimulant compound as a ratio to the stimulant efficacy determined for (+)-amphetamine.

*Locomotor depressant effects.* The potency and efficacy of the compounds were estimated for the 30-minute time period in which maximal depression occurred at the lowest dose. The mean average total distance traversed (cm/10 min) for that period was fit to a linear function of log₁₀ dose of the descending portion of the dose-effect curve. The ID₅₀ was the dose producing ½ maximal depressant activity, where maximal depression = 0 cm/30 minutes. Efficacy was the ratio of maximal depressant effect to maximum possible depression for each compound (mean average total distance of the control group minus the lowest mean total distance, expressed as a ratio to the control group total distance).

*H*_{*1*} *antihistamine interaction studies.* The potential for each compound to interact with H₁ antihistamines was determined by testing whether a known H₁ antihistamine, triprolidine, produced a dosage shift in the observed stimulant or depressant effects of each sympathomimetic compound. Triprolidine was used as an example of the class of H₁ antihistamines. Triprolidine (at 0.01, 0.1, 1.0, or 25 mg/kg) or saline was injected 20 minutes prior to administering one of three doses of each sympathomimetic compound. The mice were then immediately placed in the activity-testing apparatus for a 2 hour session. Doses of the test compound were selected from the ascending or descending portion of the dose-effect curve determined from the compound-alone studies. Eight mice were tested for each triprolidine/sympathomimetic combination.

*Statistical analysis* Time course data for each compound were considered in 2-way analyses of variance with dose as a between-group space and time as a within-group factor. The dose-effect data were considered in 1-way analysis of variance, and planned individual comparisons were conducted between each dose and the saline control group. Interaction studies were considered in 2-way analyses of variance, with Pretreatment and Test dose as the factors.

Two-way analyses of variance were conducted on horizontal distance traveled using dose as a between-subject factor and time as a within-subject factor. Only (+)-amphetamine exhibited a significant dose- and time-effect, with an interaction of dose and time (all Fs > 2.7; all p values < 0.01).

### Results:

The effects of the tested compounds on locomotor activity are summarized in Table 5.

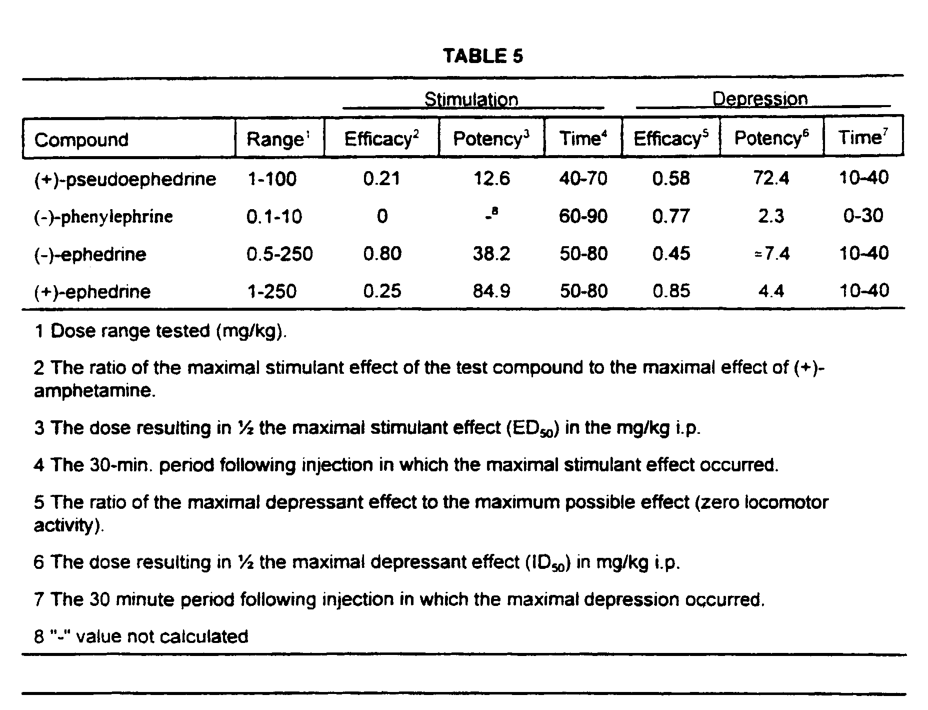

### Locomotor stimulant effects

### Time course

Mice injected with (+)-amphetamine (control) showed a dose- and time-dependent increase in the distance traversed within 10 minutes following injection. The peak stimulant effects occurred during the first 30 minutes following 2.5 mg/kg and continued for at least 60 minutes. The ED₅₀ for (+)-amphetamine was 1.3 mg/kg.

Dose dependent increases in locomotor activity of slower onset and longer duration were evident following (-)-ephedrine. (-)-Ephedrine resulted in increased locomotion within 40 minutes following 50 to 100 mg/kg, with peak effects occurring 50-80 minutes following injection and diminishing thereafter. A small increase in locomotor activity was evident following 100 mg/kg (+)-ephedrine, although most doses of this compound were without effect or depressed locomotor activity. Figure 1 summarizes these results, illustrating that (-)-ephedrine had a greater stimulant effect on the central nervous system than (+)-ephedrine. Little or no stimulant effects were evident within 2 hours following (+)-pseudoephedrine.

The potency of these compounds on locomotor stimulant effects are summarized above in Table 5.

### Locomotor depressant effects

*Time course.* Treatment with (+)-pseudoephedrine and to a lesser extent, (+)-ephedrine, did result in some dose-dependent locomotor depression within 10 to 20 minutes following injection. (Figure 2, Table 5) These effects lasted from 20 minutes to 2 hours or more, depending upon dose and compound.

*Depressant efficacy*/*potency*. The potency and efficacy for locomotor depressant effects of these compounds are also summarized in Table 5 above. Figure 2 shows the dose-response relationshipsfor locomotordepressant effects of the sympathomimetics for the time period in which the maximal depressant effects were first observed as a function of dose. The maximal depressant effect was estimated as the difference between the control group mean and the mean of the dose group with lowest locomotor activity. The maximum possible effect was assumed to be equivalent to the mean of the control group.

### Triprolidine interactions

*Triprolidine alone.* When injected immediately prior to testing, doses of triprolidine from 0.25 to 25 mg/kg failed to affect horizontal distance during the 2-hour test period. Dose-dependent depression of locomotion was observed following 50 and 100 mg/kg triprolidine, beginning within 10-minutes following injection and lasting for 30 to 40 minutes. A separate one-way analysis of variance on average distance/10 minutes for the period 0-30 minutes following injection suggested a significant dose main effect where F(8,102) = 7.7, p<0.001, although individual comparisons of dose groups with control in that analysis verified that significant effects of triprolidine were restricted to the 50 and 100 mg/kg doses (ps<.01).

*Triprolidine interactions.* Figures 3 and 4 show that triprolidine pretreatment stimulates mice treated with (+)-pseudoephedrine and (-)-ephedrine. No such central nervous system stimulation is observed in mice pretreated with triprolidine and then treated with (+)-ephedrine. The central nervous system stimulation is dose-responsive. After pretreatment with 0.01, 0.1, or 1.0 mg/kg triprolidine, increased horizontal movement is observed in mice receiving (+)-pseudoephedrineand (-)-ephedrine, but not (+)-ephedrine. Thus, no drug interaction is apparent between (+)-ephedrine and an antihistamine such as triprolidine. Combination therapies including coadministration of decongestants and antihistamines are a significant part of the upper respiratory market, so this lack of drug interaction is commercially and therapeutically useful.

### Conclusion

(+)-Ephedrine causes less central nervous system stimulation and has less potency 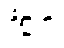 depression than does 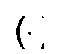 -ephedrine. Moreover, combining triprolidine and (+)-ephedrine did not give rise to any negative drug interactions such as central nervous system stimulation or depression. Hence, (+)-ephedrine does not appear to interact with H₁ histamine receptors, and may be used in combination with other drugs, particularly H₁ antihistamines such as triprolidine.

### EXAMPLE 3

### Decongestant Activity Of (+)-Ephedrine

A study was conducted comparing the decongestant activity of (+)-ephedrine, (+)-pseudoephedrine,(-)-phenylephrine and (-)-ephedrine in normal and histamine-challenged rats at various dosages.

### Experimental Protocol

The method used was based on one reported by Lung for the measurement of nasal airway resistance. 104 ACTA OTOLARYNGOL (Stockholm) 526 (1987). Sprague Dawley rats (weight range 247-365 gram) were anesthetized with sodium pentobarbital intra-peritoneally(50 mg/kg). Rats were placed on a heating pad, in a V trough, dorsal side down. A tracheotomy was performed and a tracheal cannula was positioned, tied and left open to room air. A cannula was placed into the superior part of the trachea and was advanced till lodged in the posterior nasal opening. Normal saline (0.5 ml) was injected into the nasal cannula to confirm position as well as to moisten the nasal mucosa. After nasal cannulation was confirmed the cannula was tied in place with a suture placed around the trachea. Excess fluid was expelled from the nasal airway with a short (2-3 second) air flow via the nasal cannula. Additionally, in studies correlating blood pressure changes to those in the nasal airway pressure, a cannula was positioned in the internal carotid artery (PE. 50) and connected to a multipen Grass recorder (Grass Instruments, Quincy, MA) using a pressure transducer (Isotec®, Quest Medical, Allan, TX).

Nasal airway pressure was measured using a Validyne pressure transducer (Validyne Engineering, Northridge, CA) with a 2.25 cm H₂O range membrane connected to a multipen recorder (Grass Instruments, Quincy, MA). Air was passed through an in-line direct measure flow meter (Gilmont Instruments, Barrington, IL) connected to the nasal opening cannula. Pressure was measured in the line with a constant flow rate (150 ml/min) of air. Drugs were directly injected into the jugular vein using a 30 gauge needle. All injections were of a constant 0.1 ml volume. In the congestion challenged groups congestion was achieved by an intranasal administration of histamine (50 mM, 0.02 ml/nostril). The histamine was expelled after 2 minutes with a short nasal cannula airflow and subsequent enantiomeric drug doses were directly injected into the jugular vein. The dosage chosen for each drug was the amount needed to increase the mean arterial pressure (MAP) of the animal by 10%; this dosage served as the "100%" dose for the initial nasal airway studies. The dose causing a 10% increase in MAP for dogs is provided in Table 6. Mild anxiety was observed in dogs given a 15 mg oral dosage. The dosage required for rats was calculated by proportionately adjusting the experimentally determined dog dosage to account for the lesser weight of the rats. These absolute dosages for the rat are provided in Table 6.

**TABLE 6**

| **Dosage of Enantiomer** | | |
|---|---|---|
| **Drug Name** | **Dog (µg/kg)**^{**a**} | **Rat (µg)**^{**b**} |
| (+)-pseudoephedrine | 200 | 60 |
| (-)-phenylephrine | 10 | 3-5 |
| (-)-ephedrine | 100 | 30 |
| (+)-ephedrine | 200 | 60 |

| | | |
|---|---|---|
| ^{a} Experimentally determined dosage required to raise MAP by 10%. | | |
| ^{b} Calculated from the dog dosage by proportionately decreasing the dosage to account for the lesser weight of the rat. | | |

Hence, about twice as much (+)-ephedrine, relative to (-)-ephedrine, can be administered before a 10% increase in blood pressure is observed.

Two investigations were performed on rats.
**Investigation 1:** A comparison was made of the effect of the different enantiomers on nasal airway resistance prior to and following histamine congestion. The amount of drug required to raise the mean arterial pressure by 10% was chosen as the "100% dose" for these decongestant studies. *See* Table 6. Control changes in nasal airway resistance were obtained by recording nasal airway resistance prior to and following this 100% dose. In a test group of rats the 100% dose was injected into the jugular vein two minutes after nasal airway congestion was produced by introduction of 0.02 ml/nostril of 50 mM histamine into the nasal airway. Nasal airway resistance was thus increased after the histamine challenge and the effect of administering an enantiomer on this histamine-induced airway resistance was observed.
**Investigation 2:** A comparison of the effect on nasal airway resistance of various doses of each enantiomer was made to determine an effective dose of the enantiomer. The dosages tested were 75%, 50%, 25%, 10% and 5% of the "100%" enantiomerdosage required to increase the mean arterial pressure 10%. Changes in nasal airway resistance were obtained by comparing pre-enantiomer injection nasal airway resistance with decreases in nasal airway resistance following jugular vein injection of the enantiomer. Five rats were tested at each dose for each of the enantiomers.
**Investigation 3:** A 75% dose of enantiomer (Table 7) was tested following treatment with 50 mM histamine (0.02 ml/nostril).

**Table 7**

| **Drug** | **75% Dosage (µg/kg i.v.)** |
|---|---|
| (+)-pseudoephedrine | 150 |
| (-)-ephedrine | 75 |
| (-)-phenylephrine | 7.5 |
| (+)-ephedrine | 150 |

Blood pressure was monitored. Effects on airway resistance and blood pressure of each of the eight enantiomer were evaluated at the 75% dose prior to and following histamine in five rats for each enantiomer and each histamine condition.

### Results

### Investigation 1:

Figure 5 summarizes the decrease in nasal airway resistance followed by each enantiomer prior to and following a histamine-induced nasal congestion. Each drug gave rise to a significant decrease in nasal airway pressure, relative to control, in non-histamine-challenged rats (Table 8). While the control for (-)-phenylephrinewas significantly different from the other controls, this difference in control levels did not translate into a difference caused by administration of the drug. Rats treated with (+)-ephedrine showed a significantly greater reduction in airway passage resistance than did rats with (-)-ephedrine.

**TABLE 8**

| **Drug** | **Control*** | **Post Drug*** | **% Change** | **pValue‡** |
|---|---|---|---|---|
| (+)-ephedrine | 8 ± 0.6 | 7 ± 0.9 | -21.4 ± 5.4 | 0.008 |
| (+)-pseudoephedrine | 9 ± 0.5 | 7 ± 0.9 | -21.3 ± 7.6 | 0.015 |
| (-)-phenylephrine | 5 ± 0.2 | 4 ± 0.2 | -20.3 ± 4.3 | 0.010 |
| (-)-ephedrine | 7 ± 0.8 | 6 ± 0.5 | -14 ± 3.4 | 0.034 |

| | | | | |
|---|---|---|---|---|
| * in mm H₂O. | | | | |
| ‡paired t test | | | | |

In the histamine-challenged rats, administration of each drug again showed a significant decrease in nasal passage pressure. The results are set forth in Table 9 and are summarized in Figure 5.

**TABLE 9**

| **Drug** | **Control*** | **t test p Value** | **Post* Histamine** | **t test pValue** | **Post* Drug** | **%Change** |
|---|---|---|---|---|---|---|
| (+)-ephedrine | 8.0 ± 0.6 | 0.01 | 10.2 ± 0.5 | 0.002 | 8.2 ± 0.3 | -19.4 ± 2.1 |
| (+)pseudo-ephedrine | 6.6 ± 0.6 | 0.1 | 9.3 ±1.8 | 0.001 | 6.1 ±1.5 | -36.7 ±2.7 |
| (-)-phenyl-ephrine | 7.5 ±0.5 | 0.04 | 13.2 ±2.1 | 0.05 | 10.5 ±2.1 | -22.3 ±8.5 |
| (-)ephedrine | 6.7 ±0.4 | 0.06 | 8.2 ±0.7 | 0.007 | 6.4 ±0.6 | -21.8 ±3.5 |

| | | | | | | |
|---|---|---|---|---|---|---|
| * mm water. | | | | | | |

These results indicate that all three tested enantiomers cause a significant decrease in nasal airway pressure in both non-histamine challenged- and histamine-challenged rats. Each enantiomer is therefore an effective decongestant (Figure 5).

### Investigation 2:

Table 10 summarizes the mean nasal airway pressure of different enantiomer dosages ranging from 5%, 10%, 25%, and 50% of the dose that produced a 10% change in resting mean arterial pressure (the "100%" dose). The standard error of the mean is also provided.

**TABLE 10**

| **Mean Decrease in Nasal Airway Pressure With Variable Enantiomer Dosages*** | | | | |
|---|---|---|---|---|
| **Drug** | **5%** | **10%** | **25%** | **50%** |
| (+)-ephedrine | -2.6 ± 1.0 | 3.0 ± 1.0 | -0.1 ± 1.4 | -1.0 ± 3.3 |
| (+)-pseudoephedrine | -3.8 ± 2.8 | -6.8 ± 3.6 | -13.5 ± 4.3 | -16.1 ± 2.4 |
| (-)-phenylephrine | -1.6 ± 0.9 | -4.8 ± 0.8 | -12.1 ± 2.3 | -5.2 ± 1.6 |
| (-)-ephedrine | -1.0 ± 0.8 | -2.5 ± 1.5 | -3.6 ± 1.9 | -1.9 ± 2.0 |

| | | | | |
|---|---|---|---|---|
| *percent enantiomer dose that increased the canine resting mean arterial pressure 10%. | | | | |

### Investigation 3:

At the 75% dose, (+)- and (-)-ephedrineproduced approximatelythe same efficacious effect on normal airway decongestion, however, (-)-ephedrine caused a slightly greater (though significant) increase in blood pressure than (+)-ephedrine. These results are set forth in Figures 6 and 7.

## Claims

1. A pharmaceutical composition comprising a therapeutically effective amount of ephedrine or a pharmaceutically acceptable salt thereof and a pharmaceutically acceptable carrier,
wherein the ratio of (+)-ephedrine to (-)-ephedrine is 80:20 or greater.

2. A pharmaceutical composition according to Claim 1 ,
wherein the pharmaceutically acceptable salt is selected from a hydrochloride, bitartrate, tannate, sulfate, stearate or citrate.

3. A pharmaceutical composition according to anyone of Claims 1 or 2, wherein the ratio of (+)-ephedrine to (-)-ephedrine is greater than 90:10.

4. A pharmaceutical composition according to anyone of Claims 1 to 3, wherein the ratio of (+)-ephedrine to (-)-ephedrine is greater than 95:5.

5. A pharmaceutical composition according to anyone of Claims 1 to 4, wherein the ratio of (+)-ephedrine to (-)-ephedrine is greater than 99:1.

6. A pharmaceutical composition according to anyone of Claims 1 to 5 , wherein the dosage of (+)-ephedrine is in the range of 0,1 µg to 50 mg per kg body-weight.

7. A pharmaceutical composition according to anyone of Claims 1 to 6, wherein the dosage of (+)-ephedrine is in the range of 1 µg to 10 mg per kg body-weight.

8. A pharmaceutical composition according to anyone of Claims 1 to 7, wherein the dosage of (+)-ephedrine is in the range of 50 µg to 500 µg per kg body-weight.

9. A pharmaceutical composition according to anyone of Claims 1 to 8, **characterized by** a supplementary active ingredient.

10. Use of ephedrine or a pharmaceutically acceptable salt thereof, wherein the ratio of (+)-ephedrine to (-)-ephedrine is 80:20 or greater, for the preparation of a pharmaceutical composition for suppressing the appetite.

11. Use of ephedrine or a pharmaceutically acceptable salt thereof, wherein the ratio of (+)- ephedrine to (-)-ephedrine is 80:20 or greater, for the preparation of a pharmaceutical composition for reducing nasal or bronchial congestion.

12. Use of ephedrine or a pharmaceutically acceptable salt thereof, wherein the ratio of (+)-ephedrine to (-)-ephedrine is 80:20 or greater, for the preparation of a pharmaceutical composition for treating attention deficit hyperactivity disorders.

13. Use according to anyone of Claims 10 to 12, wherein the ratio of (+)-ephedrine to (-)-ephedrine is greater than 90:10.

14. Use according to anyone of Claims 10 to 13, wherein the ratio of (+)-ephedrine to (-)-ephedrine is greater than 95:5.

15. Use according to anyone of Claims 10 to 14, wherein the ratio of (+)-ephedrine to (-)-ephedrine is greater than 99:1.

16. Use according to anyone of Claims 10 to 15, wherein the pharmaceutically acceptable salt is selected from a hydrochloride, bitartrate, tannate, sulfate, stearate or citrate.

17. Use according to anyone of Claims 10 to 16, wherein the dosage of (+)-ephedrine is in the range of 0,1 µg to 50 mg per kg body-weight.

18. Use according to anyone of Claims 10 to 17, wherein the dosage of (+)-ephedrine is in the range of 1 µg to 10 mg per kg body-weight.

19. Use according to anyone of Claims 10 to 18, wherein the dosage of (+)-ephedrine is in the range of of 50 µg to 500 µg per kg body-weight.

20. Use according to anyone of Claims 10 to 19, wherein beside the ephedrine a supplementary active ingredient is incorporated.
